# EUROPÄISCHE PATENTANMELDUNG

(11) **EP 4 046 569 A1**
(43) Veröffentlichungstag der Anmeldung: **24.08.2022**
(21) Anmeldenummer: 21158069.1
(22) Anmeldetag: 19.02.2021
(51) Int. Cl.: A61B 5/00, A61B 5/145, G01N 21/17

(54) **VERFAHREN UND SENSOR ZUM BESTIMMEN EINER KONZENTRATION EINES ZIELGASES IM BLUT EINES LEBEWESENS**

(71) Anmelder: Fraunhofer-Gesellschaft zur Förderung der angewandten Forschung e.V., 80686 München (DE); Albert-Ludwigs-Universität Freiburg, 79098 Freiburg (DE)
(72) Erfinder: SCHMIDT, Katrin, 79110 FREIBURG (DE); WEBER, Christian, M.Sc., 79110 FREIBURG (DE); WÖLLENSTEIN, Jürgen, 79110 FREIBURG (DE); HASSAN, Yassine, 791110 FREIBURG (DE)
(74) Vertreter: WSL Patentanwälte Partnerschaft mbB

(57) **Zusammenfassung**

Die vorliegende Erfindung betrifft ein Verfahren zum Bestimmen einer Konzentration eines Zielgases in Blut eines menschlichen oder tierischen Lebewesens. Dazu weist das erfindungsgemäße Verfahren die Schritte auf: Erzeugen von elektromagnetischer Anregungsstrahlung mit eine Trägerfrequenz, wobei die Trägerfrequenz derart gewählt wird, dass das Zielgas und ein Absorptionsgas die Anregungsstrahlung absorbiert, Modulieren einer Amplitude oder der Trägerfrequenz der Anregungsstrahlung mit einer Modulationsfrequenz, Beleuchten einer Absorptionsstrecke superfizial von einer Haut des Lebewesens mit der Anregungsstrahlung, Erzeugen einer Schallwelle durch eine Absorption der Anregungsstrahlung in dem Absorptionsgas und Erfassen zumindest einer Amplitude oder einer Phase der Schallwelle als Maß für eine Konzentration des Zielgases auf der Absorptionsstrecke. Die Erfindung betriff zudem einen photoakustischen Sensor zum Bestimmen einer Konzentration eines Zielgases in Blut eines menschlichen oder tierischen Lebewesens.

## Beschreibung

Die vorliegende Erfindung betrifft ein Verfahren zum Bestimmen einer Konzentration eines Zielgases in Blut eines menschlichen oder tierischen Lebewesens, einen Sensor hierfür sowie die Verwendung eines solchen Sensors.

Die bei Säugetieren in dem Blut gelösten Gase Sauerstoff (O₂) und Kohlendioxid (CO₂) sind spezifisch für den momentanen Zustand der Atmung. Sie geben sofort Aufschluss über eine z.B. unzureichende Sauerstoffzufuhr oder eine verminderte Atemfrequenz. Im klinischen Bereich wird die Konzentration von Sauerstoff und Kohlendioxid typischerweise über eine Maske direkt in der ausgeatmeten Atemluft bestimmt. Wenn eine solche Messung mithilfe einer Maske nicht möglich ist oder im klinischen Alltag hinderlich ist, so kann die Konzentration der Gase auch indirekt transkutan, d.h. mittels einem Sensor über der Haut, bestimmt werden.

Bei den aus dem Stand der Technik bekannten transkutanen, nicht invasiven Messverfahren werden kompakte Elektroden auf die Haut aufgeklebt und der Gasgehalt wird elektrochemisch erfasst. Das Kalibrierintervall bei elektrochemischen Sensoren liegt maximal in einem Bereich von bis zu 12 Stunden, da die Elektroden über einen längeren Zeitraum driften. Zum Kalibrieren muss der Sensor abgenommen und durch einen anderen ersetzt werden, um anschließend extern neu kalibriert zu werden.

Noch im Forschungs- und Entwicklungsstadium befinden sich Sensoren, mit denen die CO₂-Konzentration im Blut transkutan, nicht-invasiv mithilfe eines nicht dispersiven Infrarotsensors (NDIR-Sensor) erfasst wird. Bei solchen NDIR-Sensoren sind jedoch die Kosten vergleichsweise hoch und ohne einen Referenzkanal sind auch die NDIR-Sensoren anfällig für eine Drift und müssen in regelmäßigen, vergleichsweise kurzen Zeitintervallen nachkalibriert werden.

Gegenüber dem Stand der Technik ist es daher eine Aufgabe der vorliegenden Erfindung, ein Verfahren und einen Sensor zum Bestimmen einer Konzentration eines Zielgases im Blut eines menschlichen oder tierischen Lebewesens bereitzustellen, welche, wenn sie im Einsatz sind, lange Kalibrierintervalle ermöglichen. Zudem ist es eine Aufgabe der vorliegenden Erfindung, ein Verfahren zum Bestimmen einer Konzentration eines Zielgases im Blut eines Lebewesens sowie einen Sensor hierfür bereitzustellen, die geringere Kosten als aus dem Stand der Technik bekannte Verfahren und Sensoren verursachen.

Zumindest eine der zuvor vorgenannten Aufgaben wird durch ein Verfahren zum Bestimmen einer Konzentration eines Zielgases in Blut eines menschlichen oder tierischen Lebewesens gemäß dem unabhängigen Anspruch 1 gelöst. Dazu weist das erfindungsgemäße Verfahren die Schritte auf: Erzeugen von elektromagnetischer Anregungsstrahlung mit eine Trägerfrequenz, wobei die Trägerfrequenz derart gewählt wird, dass das Zielgas und ein Absorptionsgas die Anregungsstrahlung absorbiert, Modulieren einer Amplitude oder der Trägerfrequenz der Anregungsstrahlung mit einer Modulationsfrequenz, Beleuchten einer Absorptionsstrecke superfizial von einer Haut des Lebewesens mit der Anregungsstrahlung, Erzeugen einer Schallwelle durch eine Absorption der Anregungsstrahlung in dem Absorptionsgas und Erfassen zumindest einer Amplitude oder einer Phase der Schallwelle als Maß für eine Konzentration des Zielgases auf der Absorptionsstrecke.

Die grundlegende Idee der vorliegenden Erfindung ist es, die Konzentration eines Zielgases im Blut eines menschlichen oder tierischen Lebewesens transkutan mit einem photoakustischen Verfahren zu erfassen. Photoakustische Verfahren haben das Potenzial, ein hinreichend genaues Bestimmen einer Konzentration des Zielgases mit geringem Kostenaufwand und dennoch mit der erforderlichen Genauigkeit durchzuführen.

Bei einem photoakustischen Verfahren wird das Absorptionsgas, insbesondere ein das Absorptionsgas enthaltendes Gasgemisch, mit der elektromagnetischen Anregungsstrahlung bestrahlt, wobei die Anregungsstrahlung eine Frequenz ihres elektromagnetischen Wechselfeldes, d.h. eine Trägerfrequenz, aufweist, die gleich einer Absorptionsfrequenz des Absorptionsgases ist. Dadurch absorbiert das Absorptionsgas die Anregungsstrahlung. Es kommt zu einer Erwärmung des Zielgases und damit einhergehend zu einer thermischen Ausdehnung des Absorptionsgases bzw. des das Absorptionsgas enthaltenden Gasgemisches.

Damit aus der Beleuchtung des Absorptionsgases mit der Anregungsstrahlung Rückschlüsse auf die Konzentration des Probengases gezogen werden können, wird die Anregungsstrahlung derart moduliert, dass die thermische Ausdehnung des Probengases periodisch erfolgt und somit eine Schallwelle innerhalb des Gases entsteht.

In einer Ausführungsform der Erfindung liegt die Modulationsfrequenz in einem Bereich von 200 Hz bis 20 kHz.

Durch Erfassen der Amplitude oder der Phase der so generierten Schallwelle kann auf eine Eigenschaft des Absorptionsgases, insbesondere auf seine Konzentration, rückgeschlossen werden. Dabei dient die erfasste Amplitude und/oder Phase der Schallwelle als Maß für die Konzentration des Absorptionsgases.

Als physiologischer Vorgang diffundieren Gase aus dem Blut von Tieren oder Menschen durch die Haut hindurch. So weist die nähere Umgebung der Haut superfizial davon eine für die Konzentration der Zielgase im Blut, insbesondere von Kohlenstoffdioxid, charakteristische Konzentration auf. Aus der Konzentration des Zielgases superfizial zu der Haut lässt sich auf die jeweilige Konzentration des Zielgases im Blut des Lebewesens rückschließen.

In einer Ausführungsform der Erfindung weist die Absorptionsstrecke einen Abstand von der Haut des Lebewesens von 5 cm oder weniger auf. Aufgrund des zugrundeliegenden physiologischen Diffusionsprozesses wird die Konzentration der Zielgase aus dem Blut des Lebewesens mit zunehmendem Abstand von der Haut des Lebewesens weniger charakteristisch. Entscheidend für die Ausführung des erfindungsgemäßen Verfahrens ist, dass die Absorptionsstrecke so superfizial von der Haut angeordnet wird, dass das Zielgas aus dem Blut in den Bereich der Absorptionsstrecke diffundiert.

Grundsätzlich besteht die Möglichkeit, dass das Zielgas als Absorptionsgas auf der Absorptionsstrecke die Anregungsstrahlung absorbiert und die daraus resultierende Schallwelle, die sich in dem Zielgas ausbreitet, erfasst wird. In einer solchen Ausführungsform der Erfindung sind das Zielgas und das Absorptionsgas identisch.

In einer solchen Ausführungsform ist zudem die Amplitude der generierten und erfassten Schallwelle direkt proportional zur Konzentration des Zielgases im Bereich der Absorptionsstrecke und damit auch von der Konzentration des Zielgases im Blut des Lebewesens abhängig. Eine hohe Konzentration an Zielgas auf der Absorptionsstrecke führt zu einer Schallwelle mit großer Amplitude. Sinkt die Konzentration, so verringert sich die Amplitude der Schallwelle.

In einer Ausführungsform der Erfindung ist das Absorptionsgas in einem abgeschlossenen Detektionsvolumen einer Detektionskammer eingeschlossen. Die Absorptionsstrecke durchläuft zudem außerhalb des Detektionsvolumens ein Absorptionsvolumen, wobei das Zielgas durch die Haut des Lebewesens in das Absorptionsvolumen diffundiert. In einer solchen Ausführungsform ist die Absorptionsstrecke zweigeteilt. Zum einen durchläuft sie das Absorptionsvolumen, in das das Zielgas aus der Haut des Lebewesens diffundiert und zum anderen das abgeschlossene Detektionsvolumen mit dem Absorptionsgas in der Detektionskammer. Das Absorptionsvolumen ist in einer Strahlrichtung der Anregungsstrahlung vor dem Detektionsvolumen angeordnet.

Dabei enthält die Detektionskammer eine vorgegebene und konstante Konzentration des Absorptionsgases. Erfasst wird zumindest die Amplitude oder die Phase der Schallwelle, welche aufgrund der Absorption der Anregungsstrahlung in dem Absorptionsgas in der Detektionskammer erzeugt wird.

Zumindest die Amplitude oder die Phase der Schallwelle ist jedoch abhängig von der Leistung der Anregungsstrahlung, welche das Absorptionsgas in der Detektionskammer erreicht. Da das Absorptionsvolumen in der Strahlrichtung der Anregungsstrahlung vor der Detektionskammer angeordnet ist, führt jede Absorption der Anregungsstrahlung in dem Absorptionsvolumen zu einer Abschwächung der Leistung der Anregungsstrahlung, welche das Absorptionsgas in dem Detektionsvolumen erreicht. Die damit einhergehende Verringerung der Amplitude der in der Detektionskammer erfassten Schallwelle oder Änderung ihrer Phase ist dann ein Maß für die Konzentration des Zielgases in dem Absorptionsvolumen.

Ein Vorteil dieser Ausführungsform liegt darin, dass die Absorption durch das Zielgas in dem Absorptionsvolumen zu einer Signalverringerung oder allgemein -veränderung ausgehend von einem durch das Absorptionsgas in der Detektionskammer definierten Niveau führt. Damit ist dieses Verfahren gegenüber kleinen Konzentrationsänderungen des Zielgases in dem Absorptionsvolumen sensitiv. Die Amplitude der Schallwelle ist umgekehrt proportional zu der Konzentration des Zielgases in dem Absorptionsvolumen.

In einer Ausführungsform, in der das Zielgas und das Absorptionsgas räumlich voneinander getrennt sind, können das Zielgas und das Absorptionsgas voneinander verschiedene Gase sein, solange diese bei der Trägerfrequenz der Anregungsstrahlung absorbierend sind. In einer anderen Ausführungsform der Erfindung sind aber auch bei einer solchen Ausgestaltung das Zielgas und das Absorptionsgas identisch.

In einer Ausführungsform, in welcher das Absorptionsgas in einer abgeschlossenen Detektionskammer eingeschlossen ist und die Absorptionsstrecke darüber hinaus über ein Absorptionsvolumen für das Zielgas verfügt, genügt es, wenn die Absorptionsstrecke in den Bereich des Absorptionsvolumens einen Abstand von der Haut des Lebewesens von 5 cm oder weniger aufweist.

In einer Ausführungsform umfasst das erfindungsgemäße Verfahren darüber hinaus den Schritt: Berechnen einer Konzentration des Zielgases in dem Blut des Lebewesens aus dem Maß für die Konzentration des Zielgases auf der Absorptionsstrecke.

In einer Ausführungsform der Erfindung fließen in die Berechnung der Konzentration des Zielgases in dem Blut des Lebewesens physiologische Parameter wie z.B. die Diffusionsrate des Zielgases durch die Haut und konstruktive Parameter des für die Ausführung des Verfahrens verwendeten photoakustischen Sensors, wie z.B. ein Wert für das Volumen des Absorptionsvolumens, ein.

Zumindest eine der zuvor genannten Aufgaben wird auch durch einen photoakustischen Sensor zum Bestimmen eines Gehalts eines Zielgases in dem Blut eines menschlichen oder tierischen Lebewesens gemäß dem auf den photoakustischen Sensor gerichteten unabhängigen Anspruch gelöst. Dabei weist der photoakustische Sensor auf: eine Quelle, die derart ausgestaltet ist, dass die Quelle in einem Betrieb des photoakustischen Sensors elektromagnetische Anregungsstrahlung mit einer Trägerfrequenz erzeugt, wobei die Anregungsstrahlung mit einer Modulationsfrequenz amplitudenmoduliert oder frequenzmoduliert ist, ein Absorptionsvolumen, das derart ausgestaltet ist, dass sich in dem Betrieb des photoakustischen Sensors das durch eine Haut des Lebewesens diffundierende Zielgas in dem Absorptionsvolumen verteilt, wobei die Quelle und das Absorptionsvolumen derart angeordnet und ausgestaltet sind, dass in dem Betrieb des photoakustischen Sensors die elektromagnetische Anregungsstrahlung der Quelle eine Absorptionsstrecke innerhalb des Absorptionsvolumens beleuchtet, und einen Schalldetektor, wobei der Schalldetektor derart eingerichtet und angeordnet ist, dass im Betrieb des photoakustischen Sensors der Schalldetektor eine von der Anregungsstrahlung in einem Absorptionsgas, das eine Absorption bei der Trägerfrequenz aufweist, angeregte Schallwelle erfasst, wobei zumindest eine Amplitude oder Phase der Schallwelle ein Maß für eine Konzentration des Zielgases in dem Absorptionsvolumen ist.

Soweit im Folgenden Aspekte der Erfindung im Hinblick auf den photoakustischen Sensor beschrieben werden, so gelten diese auch für das zuvor beschriebene Verfahren zum Bestimmen einer Konzentration des Zielgases und umgekehrt. Soweit das Verfahren mit einem photoakustischen Sensor gemäß dieser Erfindung ausgeführt wird, so weist dieser die entsprechenden Einrichtungen hierfür auf. Insbesondere sind Ausführungsformen des photoakustischen Sensors zum Ausführen der zuvor beschriebenen Ausführungsformen des Verfahrens geeignet.

Die Quelle für die elektromagnetische Anregungsstrahlung kann in einer Ausführungsform eine inkohärente Quelle, wie z.B. eine Leuchtdiode, ein Glühstrahler oder eine Superkontinuumsquelle, oder in einer anderen Ausführungsform eine kohärente Quelle, wie z.B. ein Diodenlaser, sein.

Die Wahl der Trägerfrequenz bzw. der Wellenlänge der Anregungsstrahlung und damit der Quelle hängt davon ab, welches Zielgas aus dem Blut des Lebewesens erfasst werden soll. Es versteht sich, dass das Zielgas bei der Trägerfrequenz der Anregungsstrahlung eine Absorption zeigen muss.

Die Modulationsfrequenz wird so gewählt, dass eine Schallwelle mit einer Frequenz, die gleich zu der Modulationsfrequenz ist, mithilfe eines herkömmlichen Schalldetektors erfassbar ist. In einer Ausführungsform der Erfindung liegt die Modulationsfrequenz in einem Bereich von 200 Hz bis 20 kHz.

Es ist grundsätzlich möglich, die Amplitude der Anregungsstrahlung oder deren Frequenz zu modulieren. Entscheidend ist, dass durch die Modulation eine periodische Variation der Absorption und damit der thermischen Ausdehnung des Absorptionsgases erfolgt.

In einer Ausführungsform der Erfindung ist der Schalldetektor ein Wechseldruckwandler oder ein Wechselgasflusswandler. Ein Beispiel für einen Wechseldruckwandler ist ein Mikrofon, wobei es auf die konkrete Bauart des Mikrofons erfindungsgemäß nicht ankommt. Ein Wechseldruckwandler erfasst eine Druckänderung in dem Absorptionsgas, mit welchem der Wandler in Kontakt ist.

Ein Wechselgasflusswandler hingegen erfasst eine Änderung eines Gasflusses eines Gases, welches den Wandler umgibt. Ein Beispiel für einen Wechselgasflusswandler ist eine Messeinrichtung, die auf einer Temperaturänderung eines Drahtes beruht, wobei sich der Draht in der Schallwelle befindet.

In einer Ausführungsform der Erfindung weist der photoakustische Sensor auch eine Auswerteeinrichtung auf, wobei die Auswerteeinrichtung derart wirksam mit dem Schalldetektor verbunden ist, dass die Auswerteeinrichtung in dem Betrieb des photoakustischen Sensors ein Messsignal als ein Maß für die Konzentration des Zielgases in dem Absorptionsvolumen von dem Schalldetektor erhält und wobei die Auswerteeinrichtung derart eingerichtet und ausgestaltet ist, dass die Auswerteeinrichtung in dem Betrieb des photoakustischen Sensors aus dem Maß für die Konzentration des Zielgases in dem Absorptionsvolumen eine Konzentration des Zielgases in dem Blut des Lebewesens berechnet und ausgibt.

In einer Ausführungsform der Erfindung umfasst der photoakustische Sensor eine Detektionskammer, wobei die Detektionskammer ein abgeschlossenes, von dem Absorptionsvolumen getrenntes Detektionsvolumen aufweist, wobei das Detektionsvolumen der Detektionskammer das Absorptionsgas enthält und wobei das Absorptionsgas bei der gleichen Trägerfrequenz eine Absorption aufweist wie das Zielgas.

In einer Ausführungsform der Erfindung ist das Absorptionsvolumen bis auf eine Diffusionsöffnung von einem Gehäuse abgeschlossen, wobei durch die Diffusionsöffnung in dem Betrieb des photoakustischen Sensors das Zielgas in das Absorptionsvolumen einströmt.

In einer Ausführungsform der Erfindung ist die Diffusionsöffnung des Gehäuses mit einer für das Zielgas permeablen Membran verschlossen. Auf diese Weise kann eine Verunreinigung des Absorptionsvolumens während des Betriebs des photoakustischen Sensors verhindert werden. Eine solche Ausführungsform macht den photoakustischen Sensor insbesondere für die Verwendung im medizinischen Umfeld geeignet.

In einer Ausführungsform der Erfindung ist das Absorptionsvolumen bis auf die Diffusionsöffnung durch das Gehäuse gasdicht gegenüber der Umgebung abgeschlossen.

In einer Ausführungsform der Erfindung ist das Gehäuse biokompatibel, sodass es bedenkenlos mit dem Körper, insbesondere der Haut, des Lebewesens in Kontakt gebracht werden kann. Beispiele für solche biokompatiblen Materialien sind Edelstahl, Titan und ausgewählte Kunststoffe.

In einer Ausführungsform der Erfindung ist das Gehäuse sterilisationsfähig, sodass es im medizinischen Umfeld bedenkenlos eingesetzt und nach dem Einsatz sterilisiert werden kann. Insbesondere ist in einer Ausführungsform der Erfindung das Gehäuse in einem Autoklav sterilisationsfähig.

In einer Ausführungsform der Erfindung umfasst das Gehäuse neben dem Absorptionsvolumen auch die Detektionskammer mit dem von dem Absorptionsvolumen getrennten und nach außen abgeschlossenen Detektionsvolumen.

In einer weiteren Ausführungsform der Erfindung umfasst das Gehäuse darüber hinaus auch die Quelle. In einer weiteren Ausführungsform umfasst das Gehäuse auch die Auswerteeinrichtung.

In einer Ausführungsform der Erfindung umfasst der photoakustische Sensor eine Schnittstelle zum Anbinden der Auswerteeinrichtung an ein herkömmliches Datennetzwerk, insbesondere eine LAN- oder WLAN-Schnittstelle.

In einer Ausführungsform umfasst der photoakustische Sensor ein Display zum Anzeigen einer Konzentration des Zielgases in dem Blut des Lebewesens.

In einer Ausführungsform der Erfindung weist das Gehäuse eine Kontakteinrichtung auf, wobei die Kontakteinrichtung derart ausgestaltet und angeordnet ist, dass das Gehäuse mit der Kontakteinrichtung derart auf die Haut des Lebewesens aufsetzbar ist, sodass die Diffusionsöffnung zu der Haut hinzeigt.

In einer Ausführungsform der Erfindung bildet die Kontakteinrichtung einen geschlossen umlaufenden Ring um die Diffusionsöffnung. Bringt man diesen Ring in Kontakt mit der Haut, so kann das Zielgas durch die Diffusionsöffnung in das Absorptionsvolumen gelangen, während der Rest des Gehäuses das Absorptionsvolumen gasdicht gegenüber der Umgebung abdichtet.

In einer Ausführungsform der Erfindung umfasst die Kontakteinrichtung ein Dichtelement, wobei das Dichtelement derart ausgestaltet ist, dass mit dem Dichtelement eine im wesentlichen gasdichte Abdichtung zwischen dem Gehäuse und der Haut des Lebewesens bereitstellbar ist.

In einer Ausführungsform der Erfindung ist das Dichtelement eine um die Diffusionsöffnung geschlossen umlaufende Dichtung, insbesondere eine Dichtung aus Gummi oder Kautschuk. In einer Ausführungsform ist das Dichtelement ein selbstklebender um die Diffusionsöffnung geschlossen umlaufender Ring.

In einer weiteren Ausführungsform weist der photoakustische Sensor eine Heizeinrichtung auf, wobei die Heizeinrichtung derart angeordnet und ausgestaltet ist, dass die Heizeinrichtung in dem Betrieb des photoakustischen Sensors zumindest das Absorptionsvolumen oder die Haut des Lebewesens in der Umgebung der Diffusionsöffnung erwärmt. Ein solches Heizelement stellt eine direkte oder indirekte Erwärmung der Haut des Lebewesens bereit. Eine Erwärmung bewirkt eine erhöhte Durchblutung der Haut und des sie umgebenden Gewebes, sodass eine für die Bestimmung der Konzentration des Zielgases ideale Bedingung geschaffen wird.

Eine der zuvor vorgenannten Aufgaben wird auch durch die Verwendung eines photoakustischen Sensors in einer der zuvor beschriebenen Ausführungsformen zum Bestimmen der Konzentration des Zielgases in dem Blut des menschlichen oder tierischen Lebewesens gelöst.

In einer Ausführungsform wird bei der Verwendung des photoakustischen Sensors das Gehäuse derart auf die Haut des Lebewesens aufgesetzt, dass die Diffusionsöffnung in Richtung der Haut zeigt.

Weitere Vorteile, Merkmale und Anwendungsmöglichkeiten der vorliegenden Erfindung werden anhand der folgenden Beschreibung zweier Ausführungsformen und der dazugehörigen Figuren deutlich. In den Figuren sind gleiche Elemente mit gleichen Bezugszeichen bezeichnet.
- Figur 1: ist eine schematische Querschnittsansicht durch eine erste Ausführungsform eines photoakustischen Sensors zum Bestimmen einer Konzentration eines Zielgases in dem Blut eines Lebewesens.
- Figur 2: ist eine schematische Querschnittsansicht durch eine weitere Ausführungsform eines solchen photoakustischen Sensors.

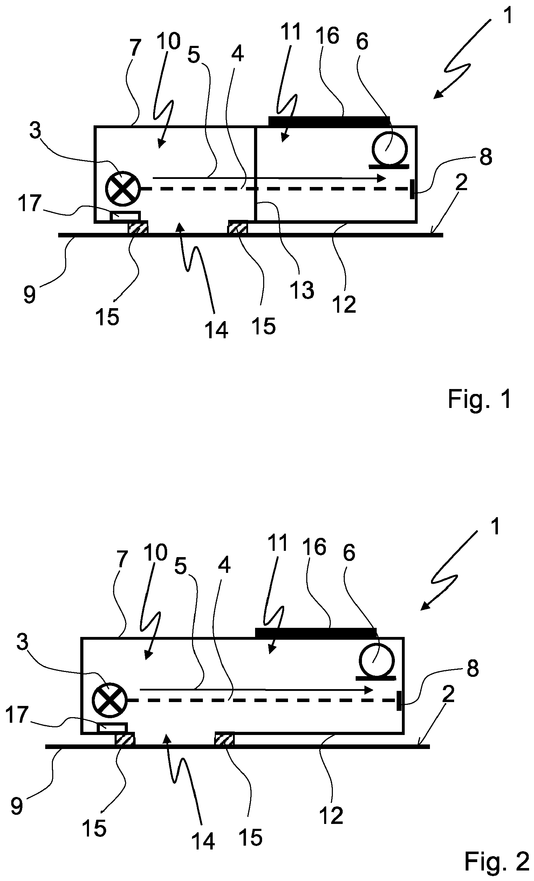

In den Querschnittsansichten der Figuren 1 und 2 sind die photoakustischen Sensoren 1 in einer seitlichen Querschnittsansicht dargestellt, wobei die photoakustischen Sensoren während ihrer Verwendung zum Erfassen der Konzentration eines Zielgases gezeigt sind. Daher ist schematisch die Hautoberfläche 2 der Haut 9 eines Lebewesens dargestellt. Auf diese Hautoberfläche 2 ist der jeweilige photoakustische Sensor 1 aufgesetzt.

Mit den in den Figuren 1 und 2 dargestellten Ausführungsformen des photoakustischen Sensors 1 sollen die Konzentration von CO₂ als Zielgas im Sinne der vorliegenden Anmeldung erfasst werden. Daher weisen die photoakustischen Sensoren 1 aus den Figuren 1 und 2 eine Quelle 3 für elektromagnetische Anregungsstrahlung mit einer Wellenlänge beispielsweise in einem Bereich um 4,3 µm auf. CO₂ absorbiert die elektromagnetische Strahlung mit dieser Trägerfrequenz.

Die von der Quelle 3 emittierte und abgestrahlte Anregungsstrahlung 4 durchläuft in dem photoakustischen Sensor 1 eine Absorptionsstrecke 5. Eine Absorption der Anregungsstrahlung in einem Absorptionsgas generierte Schallwelle wird mithilfe eines Mikrofons 6 als Schalldetektor im Sinne der vorliegenden Anmeldung erfasst.

In beiden Ausführungsformen der Figuren 1 und 2 sind das Absorptionsgas und das Zielgas jeweils identisch. Dabei könnte bei der Ausführungsform aus Figur 1 grundsätzlich auch ein von dem Zielgas verschiedenes Absorptionsgas verwendet werden, solange dieses die Anregungsstrahlung bei der gleichen Trägerfrequenz bzw. Wellenlänge absorbiert wie das Zielgas.

Die Quelle 3 und das Mikrofon 6 sind in einem Gehäuse 7 angeordnet. Damit die Anregungsstrahlung 4 nach dem Durchlaufen der Absorptionsstrecke 5 nicht eine Wand des Gehäuses 7 bestrahlt und dort eine zusätzliche Schallwelle generiert, ist in dem Gehäuse 7 ein Absorber 8 vorgesehen, welcher die Anregungsstrahlung 4 ohne Erwärmung und damit ohne eine thermische Ausdehnung des Materials der Wand 7, des Absorbers 8 oder des Gases absorbiert. Auf diese Weise wird ein Hintergrund- oder Störsignal aufgrund einer Interaktion der Anregungsstrahlung 4 mit dem Gehäuse 7 vermieden. Statt von dem Absorber 8 könnte die Anregungsstrahlung 4 auch von dem Gehäuse 7 absorbiert werden oder durch ein Fenster aus dem Gehäuse 7 austreten.

Bei der Ausführungsform der Figur 1 definiert das Gehäuse 7 in seinem Inneren ein Absorptionsvolumen 10 und ein von diesem abgetrenntes Detektionsvolumen 11. Das Detektionsvolumen 11 enthält das Kohlendioxid in einer vorgegebenen, unveränderlichen Konzentration. Das Detektionsvolumen 11 ist ein abgeschlossenes von dem Absorptionsvolumen 10 und von der Umgebung getrenntes und in einer Detektionskammer 12 eingeschlossenes Volumen. Zur Abtrennung des Absorptionsvolumens 10 von dem Detektionsvolumen 11 ist innerhalb des Gehäuses 7 ein für die Anregungsstrahlung 4 transparentes Fenster 13 vorgesehen.

Die Absorptionsstrecke 5 teilt sich somit auf das Absorptionsvolumen 10 und das Detektionsvolumen 11 auf, wobei das Absorptionsvolumen 10 in der Ausbreitungsrichtung der Anregungsstrahlung 4 vor dem Detektionsvolumen 11 angeordnet ist.

Die Anregungsstrahlung 4 von der Quelle 3 weist eine Amplitudenmodulation mit einer Modulationsfrequenz von 700 Hz auf. Durch Absorption der Anregungsstrahlung in dem Absorptionsgas wird eine Schallwelle mit einer Schallfrequenz generiert, die gleich der Modulationsfrequenz ist.

In der Ausführungsform aus Figur 1 bildet die Detektionskammer 12 einen akustischen Resonator. Dieser Resonator weist dann, wenn er mit einem Gas, insbesondere einem das Absorptionsgas enthaltenden Gasgemisch, gefüllt ist, eine Resonanzfrequenz auf. Diese Resonanzfrequenz ist gleich der Modulationsfrequenz der Anregungsstrahlung 4 und damit der Frequenz der in dem Absorptionsgas generierten Schallwelle. Durch die Ausbildung der Detektionskammer 12 als Resonator erfährt die Schallwelle eine Amplitudenüberhöhung, welche wiederum das Signal-Rausch-Verhältnis verbessert.

Befindet sich in dem Absorptionsvolumen 10 kein die Anregungsstrahl 4 absorbierendes Gas, so wird in dem Detektionsvolumen 11 eine Schallwelle generiert, deren Amplitude durch die Leistung der Anregungsstrahlung 4 und die Konzentration des CO₂ in dem Detektionsvolumen 11 bestimmt ist. Die Amplitude der Schallwelle wird mithilfe des Mikrofons 6 erfasst.

Da die normale Umgebungsluft immer auch CO₂ enthält, wird, wenn das Absorptionsvolumen 10 der Umgebungsluft ausgesetzt ist, ein gewisser Anteil der Anregungsstrahlung 4 von dem in der Umgebungsluft enthaltenen CO₂ absorbiert. Durch die Absorption in dem Absorptionsvolumen 10 reduziert sich die für die Anregung in dem Detektionsvolumen 11 bereitstehende Leistung der Anregungsstrahlung 4 und die aufgrund der Absorption in dem Detektionsvolumen 11 entstehende Schallwelle verringert ihre Amplitude.

Das Gehäuse 7 des photoakustischen Sensors 1 verfügt im Bereich des Absorptionsvolumens 10 über eine Diffusionsöffnung 14, durch die Gas in das Absorptionsvolumen 10 einströmen kann. In der dargestellten Ausführungsform ist die Diffusionsöffnung 14 ringförmig von einem selbstklebenden Film 15 umgeben. Auf dem selbstklebenden Film 15 wird der photoakustische Sensor 1 auf die Hautoberfläche 2 der Haut 9 des Lebewesens aufgesetzt, wobei die selbstklebende Folie 15 eine Abdichtung des Gehäuses und damit des Absorptionsvolumens 10 gegenüber der Hautoberfläche bereitstellt.

Diffundiert nun durch physiologische Prozesse das Zielgas, d.h. CO₂, aus dem Blut des Lebewesens durch die Haut 9 in den Bereich der Diffusionsöffnung 14, so gelangt das CO₂ in das Absorptionsvolumen 10. Dort absorbiert das CO₂ die Anregungsstrahlung 4 und verringert die Leistung der Anregungsstrahlung 4, welche in das Detektionsvolumen 11 gelangt. Mit Zunahme der Konzentration von CO₂, welches aus dem Blut durch die Haut 9 in das Absorptionsvolumen 10 diffundiert, nimmt die Amplitude der von dem Mikrofon 6 erfassten Schallwelle ab oder die Phase der Schallwelle ändert sich.

In den in den Figuren 1 und 2 dargestellten Ausführungsformen weist der photoakustische Sensor 1 ein Display 16 mit einer Auswerteeinrichtung auf, wobei die Auswerteeinrichtung 16 mit dem Mikrofon 6 verbunden ist. Auf diese Weise erhält die Auswerteeinrichtung 16 während des Betriebs des photoakustischen Sensors 1 Messwerte und damit ein Maß für die Amplitude der Schallwelle von dem Mikrofon 6. Aus dem Maß für die Amplitude der generierten Schallwelle kann die Auswerteeinrichtung 16 ein Maß für die Konzentration des Zielgases in dem Absorptionsvolumen 10 berechnen. Da man die CO₂-Konzentration in der Umgebungsluft ziemlich genau abschätzen kann und der Diffusionsprozess als solcher bekannt ist, lässt sich aus der Konzentration des CO₂ in dem Absorptionsvolumen 10 ein Maß für die Konzentration des CO₂ in dem Blut des Lebewesens bestimmen.

Der photoakustische Sensor 1 weist zudem eine Heizeinrichtung 17 auf. Mit der Heizeinrichtung 17 lässt sich das Gehäuse in der Umgebung der Diffusionsöffnung 14 erwärmen. Da das Gehäuse über den sehr dünnen selbstklebenden Film 15 in thermischem Kontakt mit der Hautoberfläche 2 steht, erwärmt die Heizeinrichtung 17 während des Betriebs des photoakustischen Sensors 1 auch die Haut und das umliegende Gewebe. Durch die Erwärmung wird der Blutfluss in diesem Bereich des Lebewesens angeregt und die Konzentrationsmessung des CO₂ in dem Absorptionsvolumen 10 wird aussagekräftiger für die tatsächliche Konzentration des CO₂ im Blut.

Figur 2 zeigt eine Variante der Ausführungsform des photoakustischen Sensors 1 aus Figur 1. Der photoakustische Sensor 1 weist nur ein Absorptionsvolumen 10 auf, sodass die Absorptionsstrecke 5 vollständig innerhalb des Absorptionsvolumens 10 liegt. Ein zusätzliches, getrenntes Detektionsvolumen existiert hier nicht. In einer solchen Ausführungsform hängt die Amplitude der durch die Absorption der Anregungsstrahlung 4 in dem CO₂ erzeugten Schallwelle unmittelbar von der Konzentration des CO₂ in dem Absorptionsvolumen 10 ab. Je höher die CO₂-Konzentration in dem Absorptionsvolumen 10 ist, desto größer ist auch die Amplitude der Schallwelle. Eine solche Ausführungsform ist technisch einfacher und daher kostengünstiger zu realisieren, weist aber gegebenenfalls ein schlechteres Signal-Rausch-Verhältnis als der photoakustische Sensor 1 aus Figur 1 auf.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

Während die Erfindung im Detail in den Zeichnungen und der vorangehenden Beschreibung dargestellt und beschrieben wurde, erfolgt diese Darstellung und Beschreibung lediglich beispielhaft und ist nicht als Beschränkung des Schutzbereichs gedacht, so wie er durch die Ansprüche definiert wird. Die Erfindung ist nicht auf die offenbarten Ausführungsformen beschränkt.

Abwandlungen der offenbarten Ausführungsformen sind für den Fachmann aus den Zeichnungen, der Beschreibung und den beigefügten Ansprüchen offensichtlich. In den Ansprüchen schließt das Wort "aufweisen" nicht andere Elemente oder Schritte aus, und der unbestimmte Artikel "eine" oder "ein" schließt eine Mehrzahl nicht aus. Die bloße Tatsache, dass bestimmte Merkmale in unterschiedlichen Ansprüchen beansprucht sind, schließt ihre Kombination nicht aus. Bezugszeichen in den Ansprüchen sind nicht als Beschränkung des Schutzbereichs gedacht.

### Bezugszeichenliste

- 1: photoakustischer Sensor
- 2: Hautoberfläche
- 3: Quelle
- 4: Anregungsstrahlung
- 5: Absorptionsstrecke
- 6: Mikrofon
- 7: Gehäuse
- 8: Absorber
- 9: Haut
- 10: Absorptionsvolumen
- 11: Detektionsvolumen
- 12: Detektionskammer
- 13: Fenster
- 14: Diffusionsöffnung
- 15: selbstklebender Film
- 16: Auswerteeinrichtung
- 17: Heizeinrichtung

## Patentansprüche

1. Verfahren zum Bestimmen einer Konzentration eines Zielgases in Blut eines menschlichen oder tierischen Lebewesens mit den Schritten
Erzeugen von elektromagnetischer Anregungsstrahlung (4) mit einer Trägerfrequenz, wobei die Trägerfrequenz derart gewählt wird, dass das Zielgas und ein Absorptionsgas die Anregungsstrahlung (4) absorbiert,
Modulieren einer Amplitude oder der Trägerfrequenz der Anregungsstrahlung (4) mit einer Modulationsfrequenz,
Beleuchten einer Absorptionsstrecke (5) superfizial von einer Haut (2) des Lebewesens mit der Anregungsstrahlung (4),
Erzeugen einer Schallwelle durch eine Absorption der Anregungsstrahlung (4) in dem Absorptionsgas und
Erfassen zumindest einer Amplitude oder einer Phase der Schallwelle als Maß für eine Konzentration des Zielgases auf der Absorptionsstrecke (5).

2. Verfahren nach dem vorhergehenden Anspruch, wobei die Absorptionsstrecke (5) einen Abstand von der Haut (2) des Lebewesens von 5 cm oder weniger aufweist.

3. Verfahren nach einem der vorhergehenden Ansprüche, wobei das Zielgas Kohlenstoffdioxid (CO₂) ist.

4. Verfahren nach einem der vorhergehenden Ansprüche, wobei das Zielgas und das Absorptionsgas identisch sind.

5. Verfahren nach einem der vorhergehenden Ansprüche, wobei das Absorptionsgas in einer abgeschlossenen Detektionskammer (12) eingeschlossen ist, wobei die Absorptionsstrecke (5) außerhalb der Detektionskammer (12) und in einer Strahlrichtung der Anregungsstrahlung (4) vor der Detektionskammer (12) ein Absorptionsvolumen (10) durchläuft, wobei das Zielgas durch die Haut (2) des Lebewesens in das Absorptionsvolumen (10) diffundiert.

6. Photoakustischer Sensor (1) zum Bestimmen eines Gehalts eines Zielgases in Blut eines menschlichen oder tierischen Lebewesens, wobei der photoakustische Sensor (1) aufweist
eine Quelle (3), die derart ausgestaltet ist, dass die Quelle (3) in einem Betrieb des photoakustischen Sensors (1) elektromagnetische Anregungsstrahlung (4) mit einer Trägerfrequenz erzeugt, wobei die Anregungsstrahlung (4) mit einer Modulationsfrequenz amplitudenmoduliert oder frequenzmoduliert ist,
ein Absorptionsvolumen (10), das derart ausgestaltet ist, dass sich in dem Betrieb des photoakustischen Sensors (1) das durch eine Haut (2) des Lebewesens diffundierte Zielgas in dem Absorptionsvolumen (10) verteilt,
wobei die Quelle (3) und das Absorptionsvolumen (10) derart angeordnet und ausgestaltet sind, dass in dem Betrieb des photoakustischen Sensors (1) die elektromagnetische Anregungsstrahlung (4) der Quelle (3) eine Absorptionsstrecke (5) innerhalb des Absorptionsvolumens (10) beleuchtet, und
einen Schalldetektor (6), wobei der Schalldetektor derart eingerichtet und angeordnet ist, dass in dem Betrieb des photoakustischen Sensors (1) der Schalldetektor (6) eine von der Anregungsstrahlung (4) in einem Absorptionsgas, das eine Absorption bei der Trägerfrequenz aufweist, angeregte Schallwelle erfasst, wobei zumindest eine Amplitude oder Phase der Schallwelle ein Maß für eine Konzentration des Zielgases in dem Absorptionsvolumen (10) ist.

7. Photoakustischer Sensor (1) nach dem vorhergehenden Anspruch, wobei der photoakustische Sensor (1) eine Auswerteeinrichtung (16) aufweist, wobei die Auswerteeinrichtung (16) derart wirksam mit dem Schalldetektor (6) verbunden ist, dass die Auswerteeinrichtung (16) in dem Betrieb des photoakustischen Sensors (1) ein Messsignal mit dem Maß für die Konzentration des Zielgases in dem Absorptionsvolumen (10) von dem Schalldetektor erhält und wobei die Auswerteeinrichtung (16) derart eingerichtet und ausgestaltet ist, dass die Auswerteeinrichtung (16) in dem Betrieb des photoakustischen Sensors (1) aus dem Maß für die Konzentration des Zielgases in dem Absorptionsvolumen eine Konzentration des Zielgases in dem Blut des Lebewesens berechnet und ausgibt.

8. Photoakustischer Sensor (1) nach Anspruch 6 oder 7, wobei der photoakustische Sensor (1) eine Detektionskammer (12) aufweist,
wobei die Detektionskammer (12) ein abgeschlossenes, von dem Absorptionsvolumen (10) getrenntes Detektionsvolumen (11) aufweist,
wobei die Detektionskammer (12) das Absorptionsgas enthält und
wobei das Absorptionsgas bei der gleichen Trägerfrequenz eine Absorption aufweist wie das Zielgas.

9. Photoakustischer Sensor (1) nach einem der Ansprüche 6 bis 8, wobei das Absorptionsvolumen (10) zumindest abschnittsweise von einem Gehäuse abgeschlossen ist, wobei das Gehäuse (7) eine Diffusionsöffnung (14) aufweist, durch die in dem Betrieb des photoakustischen Sensors (1) das Zielgas in das Absorptionsvolumen (10) einströmt.

10. Photoakustischer Sensor (1) nach dem vorhergehenden Anspruch, wobei die Diffusionsöffnung (14) mit einer für das Zielgas permeablen Membran verschlossen ist.

11. Photoakustischer Sensor (1) nach einem der Ansprüche 6 bis 10, wobei das Gehäuse (7) eine Kontakteinrichtung aufweist, wobei die Kontakteinrichtung derart ausgestaltet und angeordnet ist, dass das Gehäuse (7) mit der Kontakteinrichtung auf die Haut (2) des Lebewesens aufsetzbar ist, so dass die Diffusionsöffnung (14) in Richtung der Haut (2) zeigt.

12. Photoakustischer Sensor (1) nach dem vorhergehenden Anspruch, wobei die Kontakteinrichtung einen geschlossen umlaufenden Ring um die Diffusionsöffnung (14) bildet und wobei die Kontakteinrichtung ein Dichtelement umfasst, wobei das Dichtelement derart ausgestaltet ist, dass mit dem Dichtelement eine im Wesentlichen gasdichte Abdichtung zwischen dem Gehäuse (7) und der Haut (2) des Lebewesens breitstellbar ist.

13. Photoakustischer Sensor (1) einem der Ansprüche 6 bis 12, wobei der photoakustische Sensor (1) eine Heizeinrichtung (17) aufweist, wobei die Heizeinrichtung derart angeordnet und ausgestaltet ist, dass die Heizeinrichtung (17) in dem Betrieb des photoakustischen Sensors (1) zumindest das Absorptionsvolumen (10) oder die Haut (2) des Lebewesens in einer Umgebung der Diffusionsöffnung (14) erwärmt.

14. Photoakustischer Sensor (1) nach einem der Ansprüche 6 bis 11, wobei die Quelle (3) eine thermische Quelle oder eine Leuchtdiode ist.

15. Verwendung eines photoakustischen Sensors (1) nach einem der Ansprüche 6 bis 12 zum Bestimmen der Konzentration des Zielgases in dem Blut des menschlichen oder tierischen Lebewesens.
